# EUROPEAN PATENT SPECIFICATION

(11) **EP 4 023 269 B1**
(45) Date of publication and mention of the grant of the patent: **14.08.2024**
(21) Application number: 20306701.2
(22) Date of filing: 29.12.2020
(51) Int. Cl.: A61L 31/02, A61L 31/08, A61L 31/10, A61L 31/16, A61B 17/00, A61B 17/221

(54) **IMPROVED CLOT RETRIEVAL DEVICES AND THERAPEUTIC USE THEREOF**
VERBESSERTE GERINNSELBESEITIGUNGSVORRICHTUNGEN UND THERAPEUTISCHE VERWENDUNG DAVON
DISPOSITIFS DE RÉCUPÉRATION DE CAILLOT VEINEUX AMÉLIORÉS ET LEUR UTILISATION THÉRAPEUTIQUE

(43) Date of publication of application: 06.07.2022
(73) Proprietor: Balt Extrusion, 95160 Montmorency (FR); Institut National de la Santé et de la Recherche Médicale (INSERM), 75013 Paris (FR); UNIVERSITE PARIS 13 PARIS NORD, 93430 Villetaneuse (FR); Université Paris Cité, 75006 Paris (FR); Fondation A de Rothschild, 75019 Paris (FR)
(72) Inventor: DESILLES, Jean-Philippe Mickael, 75019 PARIS (FR); MAZIGHI, Mikael, 78600 MAISONS LAFFITTE (FR); CALIGIURI, Giuseppina, 75018 PARIS (FR); NICOLETTI, Antonino, 75018 PARIS (FR)
(74) Representative: Lavoix

(56) References cited:
- WO-A1-2016/183421
- US-A1- 2020 390 456
- ZHAO Z W ET AL: "Pyrrole-Imidazole Polyamide: A Novel Gene Silencer and its Potential Use in Drug-eluting Stents", JOURNAL OF THE FORMOSAN MEDICAL ASSOCIATION, EXCERPTA MEDICA ASIA, HONG KONG, HK, vol. 109, no. 2, 1 February 2010 (2010-02-01), pages 91 - 93, XP026938501, ISSN: 0929-6646, [retrieved on 20100201], DOI: 10.1016/S0929-6646(10)60028-9

## Description

### Field of the invention

The present invention relates to clot retrieval devices and their therapeutic use.

### Technical background

Acute ischemic strokes (AIS) are the second cause of death in the world according to World Health Organization. AIS caused 6.9 million deaths in 2013 and 3.3 million deaths in 2015, becoming a major public health problem. They are the consequence of a blood stopped-circulation provoked by a thrombus in a brain artery, who leads to a lack of oxygen and nutrients in the ischemic brain area. In fact, the neuronal cells require a continuous oxygenized-blood flow otherwise they die quickly. In order to overturn the ischemic condition, it is necessary to eliminate the thrombus in order to re-establish the blood flow.

To reach this goal, two techniques are used. The first is the thrombolysis who consists in injecting in the blood circulation a drug able to dissolve the thrombus. However, the therapeutic window for this procedure is very tight: the European taskforce recommends proceeding with thrombolysis no later than 4 hours and a half after the first symptoms and in the USA this time falls to 3 hours. The issue is serious since the administration of this treatment beyond the recommended time frame can cause brain hemorrhages, which irremediably aggravates the cerebral tissue damages.

The second technique, developed in 2015, consists in the mechanical retrieval of the occluding thrombus, which is called thrombectomy. Under direct visualization of the arterial lumen, the thrombus is crossed by a guide connected to a stent retriever. The stent is then deployed and pulled; the stent meshes can then trap the thrombus and aid retrieving it throughout the arterial tree. The current indication for this strategy targets large arteries in imaging-visible area and its use is allowed only within 6 hours since the first symptoms. A successful mechanical thrombectomy can help the patients returning to an autonomous life after the intervention.

Document US2020/390456 discloses a treatment system comprising a treatment device and one or more catheters. The treatment device comprises (i) an elongated member having a proximal portion and a distal portion configured to be positioned within a blood vessel at a treatment site at or near a thrombus and (ii) a distal element coupled to the distal portion of the elongated member via a connection assembly, the distal element comprising an expandable mesh having a constrained state for delivery to the treatment site and an expanded state in which at least a portion of the mesh is configured to be in apposition with the blood vessel wall at the treatment site, wherein the distal element is configured to expand into contact with the blood vessel wall at the treatment site and anchor and/or stabilize the elongated member within the blood vessel.

Document WO2016/183421 discloses an expandable medical device comprising a therapeutic coating comprising a polydopamine coated therapeutic agent disposed on a surface of the expandable medical device. The therapeutic agent is for example selected from the group consisting of paclitaxel, everolimus, rapamycin, sirolimus, tacrolimus, heparin, diclofenac, aspirin and any combination thereof.

Nevertheless, surgeons have sometime troubles to pull out the thrombus and sometimes some thrombus pieces cannot be removed.

There is therefore a need for improving efficiency of clot retrieval devices, in particular for increasing the chances of removing the whole thrombus and reducing the risks of mechanical arterial damage.

### Detailed description of the invention

The Inventors have surprisingly found that coating an agent targeting extracellular chromatin on a clot retrieval device allows improving the capture of a thrombus. Digoxigenin and distamycin were successfully used as extracellular chromatin targeting agent.

Intracerebral thrombi indeed contain a high quantity of genetic material, organized such like a complex mesh crossing the inside of the thrombus as well as covering the thrombus surface. This genetic material probably derives from the process of "NETosis" of the neutrophils trapped inside of the thrombi. The Neutrophil Extracellular Traps (NETs) are released by activated neutrophil and contain several components of chromatin: mainly DNA, but also histones and enzymes that regulate the biology of chromatin, such as topoisomerases. The complex mesh formed by the NETs is thought to play an important role in consolidating the thrombus and anchoring it to the vascular wall of patients with AIS (*Acute Ischemic Stroke*).

The coating according to the invention thus allows the adhesion of the clot retrieval device to the biologic moiety of the thrombus, thereby improving capture of the whole thrombus. The stable capture of the chromatin can indeed guarantee the retrieval of the thrombus in one whole piece (thus reducing or avoiding its fragmentation along the retrieval pathway of the guide). Besides, the immediate and firm capture of the thrombus allows reducing the number of stent retriever and its delivery system passages through the cerebral arterial tree and hence reducing the risk of mechanical, procedure-related, arterial damage.

Targeting chromatin is particularly advantageous, since chromatin is not be present in the blood flow nor on the vascular wall, thereby avoiding saturation of the clot retrieval device through its journey from the arterial access through to the occlusion site in the blood circulation or its adhesion to the arterial wall at the site of thrombectomy.

Since digoxigenin and distamycin cannot directly adhere to the metallic surface of clot retrieval devices, an intermediate layer of polymer can be used to functionalize the surface of the clot retrieval device and render it suitable to be coated with the chromatin binding agents.

The clot retrieval device coated with a chromatin binding agent thus allows limiting the risk of micro-embolism during the removal of the thrombus as well as risk of mechanical, procedure-related, arterial damage.

A first object of the invention is thus a clot retrieval device, preferably a stent retriever, wherein at least the part of said device intended to be in contact with a clot is coated with at least one chromatin binding agent, wherein said chromatin binding agent is selected from the group consisting of digoxigenin, a digoxigenin derivative, distamycin and a distamycin derivative.

Said chromatin binding agent is coated on the part of the device intended to be in contact with a clot via at least one coating agent.

Said coating agent may be PDA or PDA-PEG bis amine.

The clot retrieval device as defined above is characterized in that at least the part of said device intended to be in contact with a clot is coated with at least one coating agent and said chromatin binding agent is linked to said coating agent.

The digoxigenin derivative may be a compound of the following formula (III) 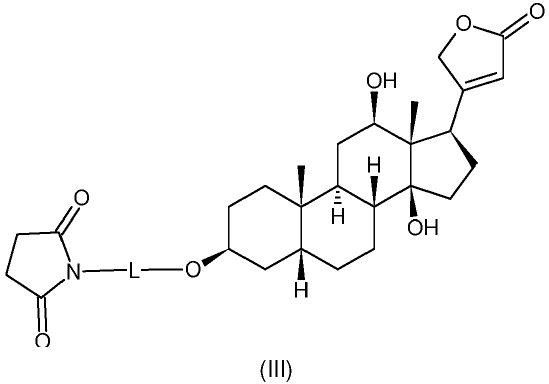 wherein L is a linker comprising at least one group selected from the group consisting of an amino, ester, amido, carboxy and hydroxyl group.

The digoxigenin derivative is preferably a digoxigenin NHS ester.

The distamycin derivative is preferably distamycin azide.

A preferred clot retrieval device as defined above is characterized in that (i) the chromatin binding agent is digoxigenin NHS ester and the coating agent is PDA PEG bis-amine or (ii) the chromatin binding agent is distamycin azide and the coating agent is PDA.

Another object of the invention is a method for producing a clot retrieval device as defined above, wherein said method comprises:
a) coating at least the part of the device intended to be in contact with a clot with at least one coating agent, and
b) contacting the part of the device coated with at least one coating agent obtained in step a) with at least one chromatin binding agent.

Step a) may comprise contacting at least the part of the device intended to be in contact with a clot with a solution comprising dopamine and, optionally, PEG bis-amine.

Another object of the invention is the use of at least one chromatin binding agent for manufacturing a clot retrieval device as defined in the claims, wherein said chromatin binding agent is selected from the group consisting of digoxigenin, a digoxigenin derivative, distamycin and a distamycin derivative.

Also disclosed is a method for removing a clot in a subject, wherein said method comprises removing said clot with a clot retrieval device as defined above or obtained by the method as defined above.

Further disclosed is a method for preventing and/or treating stroke in a patient in need thereof, wherein said method comprises removing at least one clot with a clot retrieval device as defined above or obtained by the method as defined above.

### Clot retrieval device

By "clot retrieval device comprises", it is herein meant a medical device designed to restore blood flow in patients experiencing ischemic stroke due to vessel occlusion.

### Clot and thrombus are herein synonymous.

The clot retrieval device preferably comprises a metallic surface, for example made of at least one transition metal, such as selected from the group consisting of cobalt, chromium, tungsten, nickel, iron, manganese and titanium.

The metallic surface of the clot retrieval device may for example comprise an alloy of at least two transition metals, such as for example an alloy comprising nickel and titanium (such as nitinol) or an alloy comprising cobalt and chromium.

The clot retrieval device is preferably a stent retriever.

A stent retriever is a cylindrical device consisting of a self-expanding stent mounted on a wire and deployed within a catheter.

### Chromatin binding agent

The clot retrieval device as defined above is coated with at least one chromatin binding agent.

By "chromatin binding agent", it is herein meant a compound able to bind chromatin.

The skilled person can easily determined if a given compound is able to bind chromatin. For example, the compound to be assessed is coated on a disk, for example a cobalt-chromium disk or a nitinol disk. The disk is washed, for example three times in distilled water, and then immersed in a solution comprising genetic material (in particular genomic DNA and associated proteins), at 37°C during 5 minutes. Said genetic material is for example obtained from mouse tail, for example using the "F-355L DNA Release" kit (Thermo Scientific). The disk is then thoroughly rinsed to removed unbound genetic material and immersed in a solution containing a DNA labelling marker, for example SYTOX Green, for example during 30 minutes, and washed to removed unbound DNA labelling marker. The signal from the DNA labelling marker on the disk coated with the compound to be assessed is then measured and, for example, is compared to the signal obtained using a non-coated disk. The presence of DNA on the coated disk surface indicates that the assessed compound is able to bind chromatin.

In the frame of the present invention, the chromatin binding agent is selected from the group consisting of digoxigenin, a digoxigenin derivative, distamycin and a distamycin derivative.

The chromatin binding agent may comprise an azide group or a NHS (N-Hydroxysuccinimide) group (also referred to as Su-o).

Digoxigenin is a steroid found in plants, such as *Digitalis purpurea, Digitalis orientalis* or *Digitalis lanata.*

Digoxigenin is referred under CAS number 1672-46-4.

Digoxigenin may be obtained by chemical synthesis or by purification from plants.

By "digoxigenin derivative", it is herein meant a compound derived from digoxigenin, for example by adding at least one functional group to digoxigenin, in particular for improving its linking to a coating agent.

The digoxigenin derivative may thus be obtained by chemical synthesis from digoxigenin.

The digoxigenin derivative preferably comprise a NHS group.

The digoxigenin derivative is for example a compound of the following formula (III): 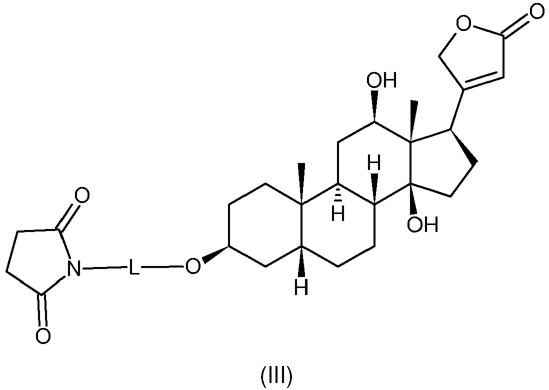 wherein L is a linker, for example a linker comprising at least one group selected from the group consisting of an amino (such as -NH- or -N((C₁-C₁₂)alkyl)-), ester (for example -C(=O)-O- or -O-C(=O)), amido (such as -C(=O)-NH-, -NH-C(=O)-, -C(=O)-N((C₁-C₁₂)alkyl)- or -N((C₁-C₁₂)alkyl)-C(=O)), and hydroxy (-O-) group.

The digoxigenin derivative of formula (III) is for example a compound of the following formula (IV): 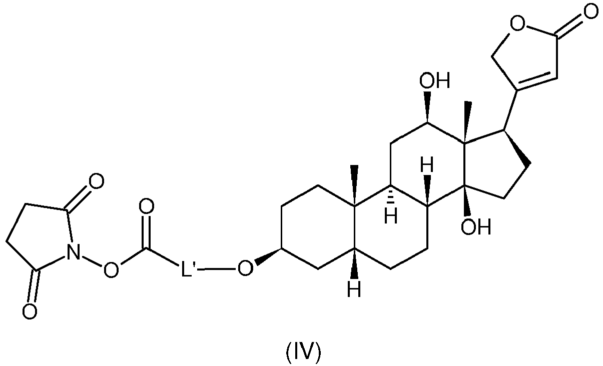 wherein L' is a linker, for example a linker comprising at least one group selected from the group consisting of an amino, ester, amido, and hydroxy group.

The digoxigenin derivative of formula (III) is preferably a compound of the following formula (V): 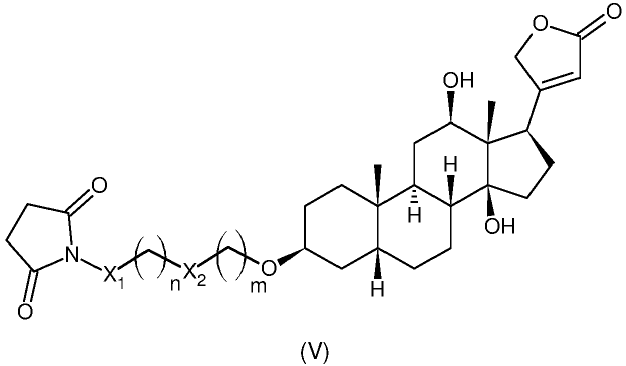
wherein X₁ and X₂ are, independently from each other, one group selected from the group consisting of: -O-, -NH-, -NR-, -O-CO-, -CO-O-, -NR-CO- and -CO-NR-, wherein R represents H or an alkyl comprising from 1 to 12 carbon atoms,
wherein n is an integer comprised from 1 to 10, for example 5, and
wherein m is an integer comprised from 1 to 5, for example 1.

Within the present application, the term "alkyl group" means: a linear or branched, saturated, hydrocarbon-based aliphatic group comprising, unless otherwise mentioned, from 1 to 12 carbon atoms, preferably from 1 to 6 carbon atoms. By way of examples, mention may be made of methyl, ethyl, n-propyl, isopropyl, butyl, isobutyl, tert-butyl or pentyl groups.

In a preferred embodiment, the digoxigenin derivative is a compound of the above formula (V), wherein X₁ is -O-CO-, X₂ is -NH-CO-, n is an integer comprised from 1 to 10, for example 5, and m is an integer comprised from 1 to 5, for example 1.

A preferred digoxigenin derivative as defined above is the compound of the following formula (VI) (also referred to as digoxigenin NHS ester, DIG-NHS or ε-(Digoxigenin-3-0-acetamido)caproic acid N-hydroxysuccinimide ester):

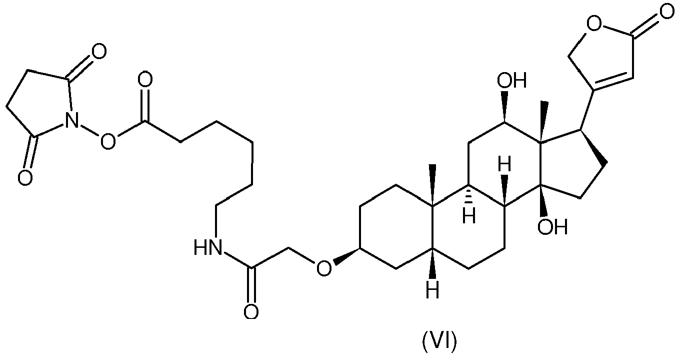

Digoxigenin and digoxigenin NHS ester of are commercially available.

Distamycin (also referred to as Distamycin A) is a pyrrole-amidine antibiotic.

Distamycin A is for example in the form of dystamycin A hydrochloride, which is referred under CAS number 6576-51-8.

Distamycin may be obtained by chemical synthesis.

Distamycin is commercially available.

By "distamycin derivative", it is herein meant a compound derived from distamycin, for example by adding at least one functional group to distamycin in particular for improving its linking to a coating agent.

The distamycin derivative may thus be obtained by chemical synthesis from distamycin.

The distamycin derivative is for example distamycin azide.

### Coating agent

A coating agent is preferably used for coating at least one chromatin binding agent as defined above on the clot retrieval device as defined above.

The coating agent is preferably a polymer or a copolymer able to adhere to a metallic surface.

The coating agent is also a compound safe for use on a medical device.

The coating agent may for example be selected from the group consisting of polydopamine (PDA) and PDA-PEG bis amine (PPba) copolymer.

Polydopamine (PDA) is a synthetic polymer made of L-DOPA (3,4-dihydroxyphenylalanine). PDA is a bio-inspired, self-assembling polymer, which shows exceptional adherence to metallic surfaces.

PDA may be obtained by polymerization of dopamine. Dopamine, a small molecule previously known for its biological role as a neurotransmitter, which combines an amine and a catechol group (which is converted into quinone by oxidation), when dissolved in an aqueous buffer at a slightly basic pH, indeed self-polymerizes into a very adherent film, on various types of substrates.

PDA-PEG bis amine (PPba) is a copolymer obtained by polymerization of dopamine and PEG (poly (ethylene glycol) bis-amine).

### Clot retrieval device coated with at least one DNA binding agent

The present invention particularly related to a clot retrieval device, wherein at least the part of said device intended to be in contact with a clot is coated with at least one chromatin binding agent.

The clot retrieval device may thus be completely or partially coated with at least one chromatin binding agent.

The "clot retrieval device", the "chromatin binding agent" and the "coating agent" are particularly as defined above in the sections of the same name.

The chromatin binding agent is thus selected from the group consisting of digoxigenin, a digoxigenin derivative, distamycin and a distamycin derivative.

The part of said device intended to be in contact with a clot is coated with one chromatin binding agent.

Alternatively, the part of said device intended to be in contact with a clot may be coated with two chromatin binding agents or at least two chromatin binding agents.

A preferred chromatin binding agent is digoxigenin, the digoxigenin derivative of formula (III) (preferably the digoxigenin derivative of formula (IV), more preferably the digoxigenin derivative of formula (V), still more preferably digoxigenin NHS ester), distamycin or distamycin azide.

The at least one chromatin binding agent is preferably coated on the part of the device intended to be in contact with a clot via at least one coating agent.

A preferred coating agent is PDA or PDA-PEG bis-amine.

The clot retrieval device as defined above is preferably characterized in that at least the part of said device intended to be in contact with a clot is coated with at least one coating agent and said at least one chromatin binding agent is linked to said coating agent, preferably covalently linked to said coating agent.

The present invention particularly relates to a clot retrieval device as defined above, wherein (i) the chromatin binding agent is digoxigenin NHS ester and the coating agent is PDA-PEG bis-amine or (ii) the chromatin binding agent is distamycin azide and the coating agent is PDA.

The clot retrieval device as defined above may be obtained by the method disclosed below.

### Method for producing a clot retrieval device coated with at least one chromatin binding agent

The present invention also relates to a method for producing a clot retrieval device as defined above, wherein at least the part of said device intended to be in contact with a clot is coated with at least one chromatin binding agent.

The method particularly comprises:
a) contacting at least the part of the device intended to be in contact with a clot with at least one coating agent, and
b) contacting the part of the device coated with at least one coating agent obtained in step a) with at least one chromatin binding agent.

The "clot retrieval device", the "chromatin binding agent" and the "coating agent" are preferably as defined above.

### Pre-step a)

The method as define above may comprise a pre-step a) before step a), comprising cleaning the part of the device to be coated with at least one chromatin binding agent, in particular at least the part of the device intended to be in contact with a clot.

This cleaning step may for example comprise:
(i) ultrasonically polishing at least the part of the device intended to be in contact with a clot, preferably in three successive baths (for example acetone, ethanol and finally distilled water), for example 10 minutes for each bath, in order to remove oxidation and organic residues, and/or
(ii) washing at least the part of the device intended to be in contact with a clot in a buffer, preferably a Tris buffer, for example three 3 times, for example during 3 minutes or each wash, preferably under stirring, for example at 400 rpm.

### Step a)

Step a) comprises coating at least the part of the device intended to be in contact with a clot with at least one coating agent.

Step a) thus allows obtaining a clot retrieval device, wherein the surface of at least the part of the device intended to be in contact with a clot is coated with said at least one coating agent. Said surface is also referred to as the "coated surface".

The coating with the coating agent is preferably obtained by direct polymerization of the coating agent onto the surface of the device.

Step a) as defined above preferably comprises contacting, preferably immersing, at least the part of the device intended to be in contact with a clot with a solution comprising dopamine and, optionally, PEG bis-amine.

Dopamine polymerize to give DPA directly onto the surface of the device.

Dopamine and PEG bis-amine are used for obtaining the copolymer DPA-PEG bis-amine by polymerization directly onto the surface of device.

In a preferred embodiment, step a) may comprise contacting (preferably immersing), preferably under stirring (for example at 400 rpm), the surface of at least the part of the device intended to be in contact with a clot with an alkaline (preferably at pH 8.5) solution comprising dopamine and, optionally PEG bis-amine, preferably in the air and in the dark, preferably at a temperature comprised between 18°C and 30°C, in particular at room temperature, and preferably for a duration comprised from 15 hours to 30 hours, in particular comprised from 17h to 24h.

Step a) preferably further comprises a rinsing step, in particular for stopping the polymerization of the coating agent. Said rinsing step may for example comprise:
- washing at least the coated part of the device with water, preferably by at least three washes in distilled water, preferably under stirring, for example during 3 minutes for each wash, and
- optionally, a brief sonication of at least the coated part of the device (preferably no more than 15 seconds), after the last wash.

Step a) thus results in a layer of the coating agent onto the surface of at least the part of the device intended to be in contact with a clot.

The layer of coating agent (in particular the PDA layer or the PDA-PEG bis -amine layer) preferably has a thickness comprised from 20 nm to 100 nm, preferably from 30 nm to 50 nm, more preferably of 45 nm.

When the coating agent is PDA, step a) may for example comprise contacting (preferably immersing) at least the part of the device intended to be in contact with a clot with a solution comprising dopamine. Said solution may for example comprise from 0.5 to 10 mg/ml of dopamine, preferably from 1 to 2 mg/ml of dopamine, preferably in a Tris buffer.

When the coating agent is PDA-PEG bis-amine, step a) may for example comprise contacting (preferably immersing) at least the part of the device intended to be in contact with a clot with a solution comprising dopamine and PEG bis-amine, preferably in a weight ratio dopamine: PEG bis-amine from 1:2 to 1:4, for example of 1:3. Said solution may for example comprise from 0,5 to 10 mg/ml of dopamine, preferably from 1 to 2 mg/ml of dopamine, more preferably 1 mg/ml of dopamine, and from 0.5 to 15 mg/ml of PEG bis-amine, preferably from 1 to 6 mg/ml of PEG bis-amine, more preferably 3 mg/ml of PEG bis-amine, preferably in a Tris buffer.

### Step b)

Step b) comprises contacting the part of the device coated with at least one coating agent obtained in step a) with at least one chromatin binding agent.

Step b) allows obtaining a device, wherein at least the part of the device intended to be in contact with a clot is coated with said at least one chromatin binding agent, by linking said at least one chromatin agent to said at least one coating agent, preferably by covalent linking.

Step b) may for example comprise contacting (preferably immersing) the part of the device coated with at least one coating agent obtained in step a) with a solution comprising at least one chromatin binding agent.

The linking between the chromatin agent and the coating agent may be direct or indirect.

By the expression "direct linking", it is herein meant that the chromatin binding agent is directly linked to the coating agent. This is for example the case when PDA-PEG bis-amine is used as coating agent and digoxigenin NHS ester as chromatin binding agent.

The part of the device coated with at least one coating agent obtained in step a) is for example contacted, preferably immersed, in a solution comprising the chromatin binding agent, for example during 30 minutes, preferably under stirring (for example at 400 rpm), at room temperature and in the dark. The solution comprising the chromatin binding agent may for example be a solution of PBS 1X or sodium bicarbonate (10mM, pH 8.3).

For example, the part of the device coated with PDA-PEG bis-amine obtained in step a) is immersed in a PBS 1X or sodium bicarbonate (10mM, pH 8.3) solution comprising from 80 µg/mL to 200 µg/mL of digoxigenin NHS ester, for example from 100 µg/ml to 150 µg/ml of digoxigenin NHS ester.

By the expression "indirect linking", it is herein meant that the chromatin binding agent is linked to the coating agent via a linker.

The surface of the device coated with the coating agent in step a), in particular the PDA coated surface, is for example modified through the fixation of a biorthogonal, copper-free click chemistry-suitable linker and the chromatin binding agent is linked to the linker by a copper-free click chemistry reaction.

Step b) may thus for example comprise:
- contacting, preferably immersing, the part of the device coated with at least one coating agent obtained in step a) with a solution comprising the linker, preferably under stirring, preferably at a temperature comprised between 18°C and 30°C, in particular at room temperature, and preferably for a duration comprised between 18 hours and 30 hours, in particular comprised between 20h and 24h, to obtain a coated surface linked to a linker,
- optionally, rinsing the coated surface linked to a linker, in particular with deionized water, and
- contacting, preferably immersing, the coated surface linked to a linker with a solution comprising at least one chromatin binding agent, wherein said chromatin binding agent comprises an azide terminal group, preferably in water, preferably at room temperature, preferably during 24 hours, for example at a concentration of the chromatin binding agent of from 0.001 µM/cm² and 200 µM/cm² of surface of the device.

The layer made of the linker may for example have a thickness comprised from 0.03 nm to 3 nm, preferably from 0.1 nm to 0.2 nm, more preferably of 0.15 nm.

The thickness of the layers made of the linker and of the chromatin-binding agent is preferably comprised from 0.5 nm to 15 nm, preferably from 1 nm to 10 nm, more preferably is of 5.6 nm.

The thickness of the coating agent layer and of the layers made of the linker and of the chromatin binding agent is preferably comprised from 20 nm to 200 nm, preferably from 10 nm to 160 nm, more preferably from 30 nm to 120 nm.

The linker is preferably an alkyne derivative, preferably a cyclooctyne derivative. The linker is thus preferably characterized by the presence of at least one triple bond, especially able to react with an azide group, in particular by click chemistry.

According to one embodiment, the linker has the formula (I-1): 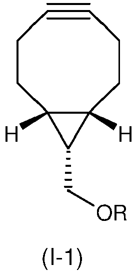 wherein R is a radical of formula -X₁-A₁-NH₂,
- X₁ being chosen from the group consisting of: -CONH-, -CO-, -CS- and - CSNH, X₁ being preferably -CONH- or -CO-, and
- A₁ being an alkylene radical comprising from 2 to 40 carbon atoms, possibly interrupted by at least one oxygen atom.

According to a preferred embodiment, the linker comprising at least one alkyne function has the following formula (I): 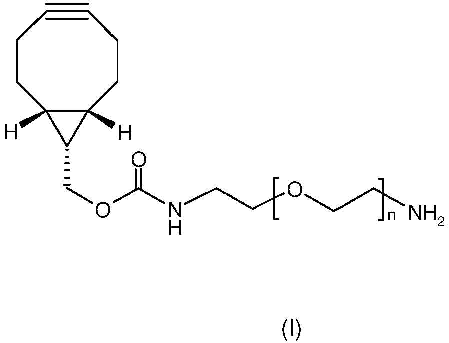 wherein n is an integer comprised between 2 and 14.

Preferably, the linker has the following formula (II): 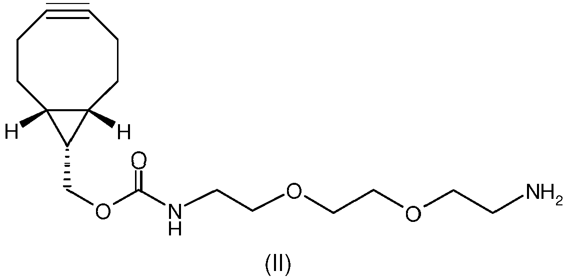 Use of at least one chromatin binding agent for manufacturing a clot retrieval device

The present invention also relates to the use of at least one chromatin binding agent for manufacturing a clot retrieval device, in particular a clot retrieval device having an improve efficacy and safety, wherein said chromatin binding agent is selected from the group consisting of digoxigenin, a digoxigenin derivative, distamycin and a distamycin derivative.

Also disclosed is the use, in particularly the *in vitro* use, of at least one chromatin binding agent to improve efficacy and safety of a clot retrieval device, wherein said chromatin binding agent is selected from the group consisting of digoxigenin, a digoxigenin derivative, distamycin and a distamycin derivative.

The "chromatin binding agent" and the "clot retrieval device" are particularly as defined above.

The chromatin binding agent is coated on the surface of at least the part of the clot retrieval device intended to be in contact with a clot, preferably via a coating agent as defined above.

### Method for removing a clot in a subject

Disclosed is also a method for removing a clot in a subject in need thereof, wherein said method comprises removing said clot with a clot retrieval device as defined above or obtained by the method as defined above.

Further disclosed is a method for preventing and/or treating stroke in a subject in need thereof, wherein said method comprises removing at least one clot with a clot retrieval device as defined above or obtained by the method as defined above.

The subject is preferably a mammalian, more preferably a human being.

The invention will be further illustrated by the following figures and examples. However, these examples and figures should not be interpreted in any way as limiting the scope of the present invention.

### Figures

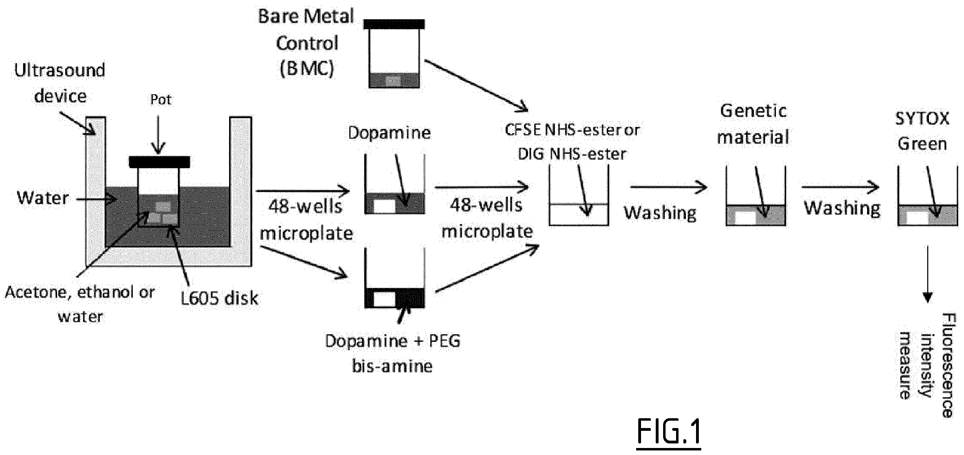

**Figure 1****:** Experimental plan for assessing chromatin binding

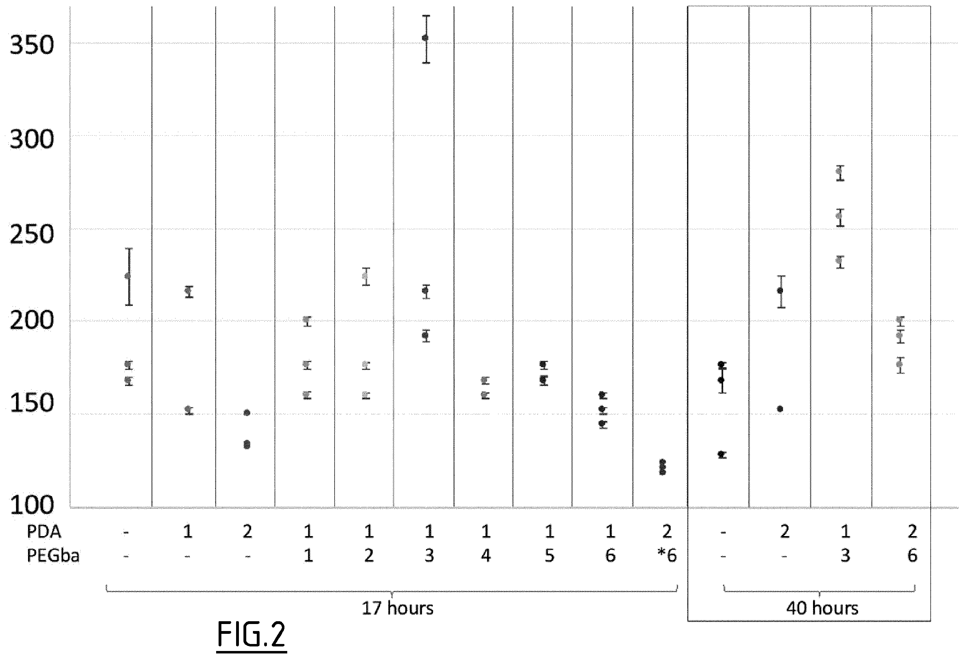

**Figure 2****:** Optimization of the copolymer for CFSE-NHS ester binding. Different polymerization time (17 or 40 hours), PDA, and PEGba concentration (mg/ml) were evaluated. Each dot represents an individual sample. Effective binding of the CFSE-NHF ester was measured by the peak±SD fluorescence on each sample in the different conditions, both at 17h and 40h of polymerization. *PEGba was used *after* (not together with) PDA). Ordinate: Fluorescence intensity peak value (A.U.).

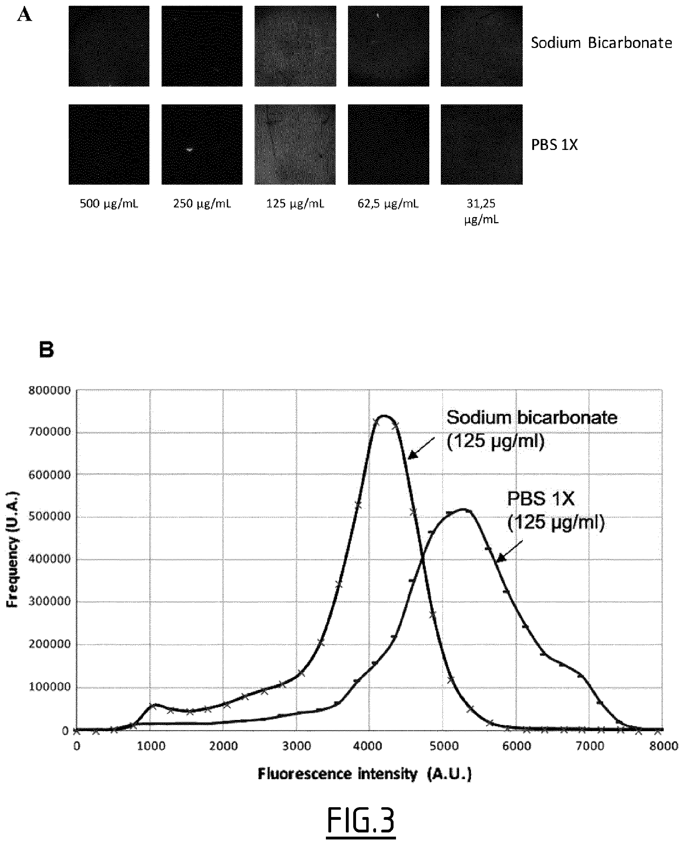

**Figure 3****:** Digital images of fluorescence intensity (A) ; Fluorescence intensity of DIG NHS ester at 125 µg/mL in PBS 1X and in sodium bicarbonate (B). The higher fluorescence intensities are both for sodium bicarbonate and PBS 1X at 125 µg/mL. Fluorescence intensities show PBS 1X has a higher fluorescence but a wider peak than sodium bicarbonate.

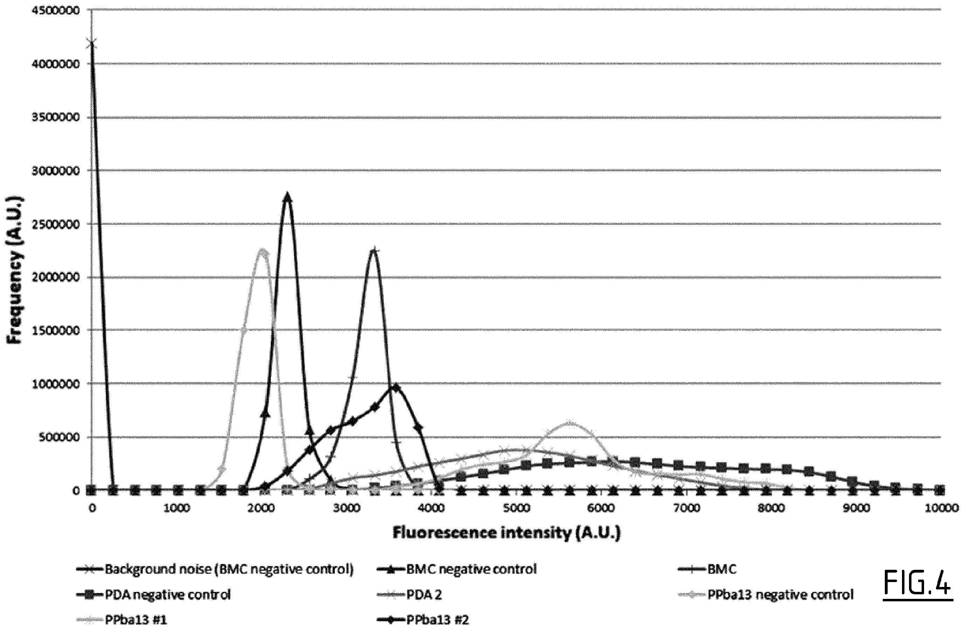

**Figure 4****:** Fluorescence intensity of BMC, PDA2, PPba13 and their respective negative controls. PPba13 disks have high fluorescence intensities values with peaks, whereas PDA2 negative control and PDA2 disk are spread along the X-axis. BMC negative control and BMC disks have narrow peaks with fewer fluorescence intensities. Ordinate: Mediane Leverage Residuals

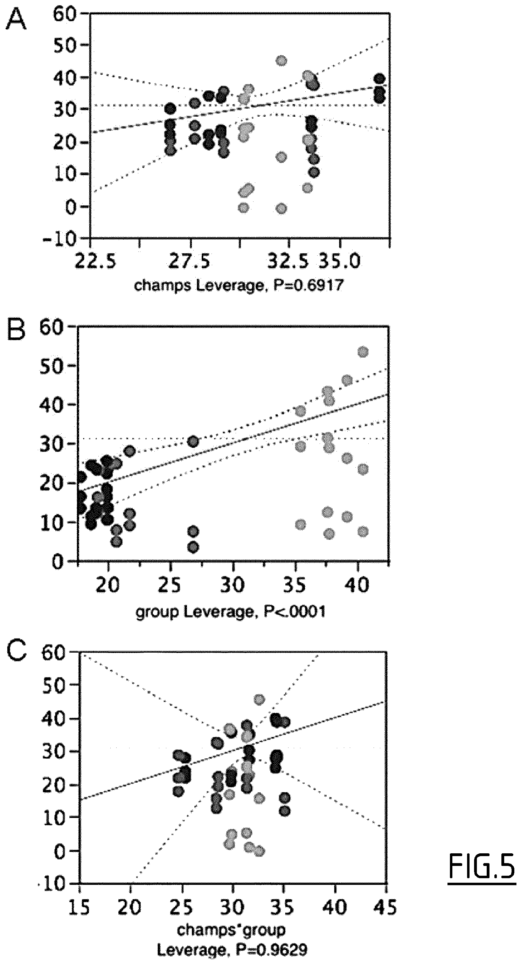

**Figure 5****:** Analysis of DNA binding by Sytox green. (A) analysis of the signal homogeneity, via the comparison of the different fields ("champs"). (B) analysis of DNA binding extent among groups. (C) Intersection of the intersection of the two variables (field and group effect). BMC values are in red, PDA values are in blue, and PPba values are in green. There is no champ effect, but a group effect, and no interdependency between champs and groups.

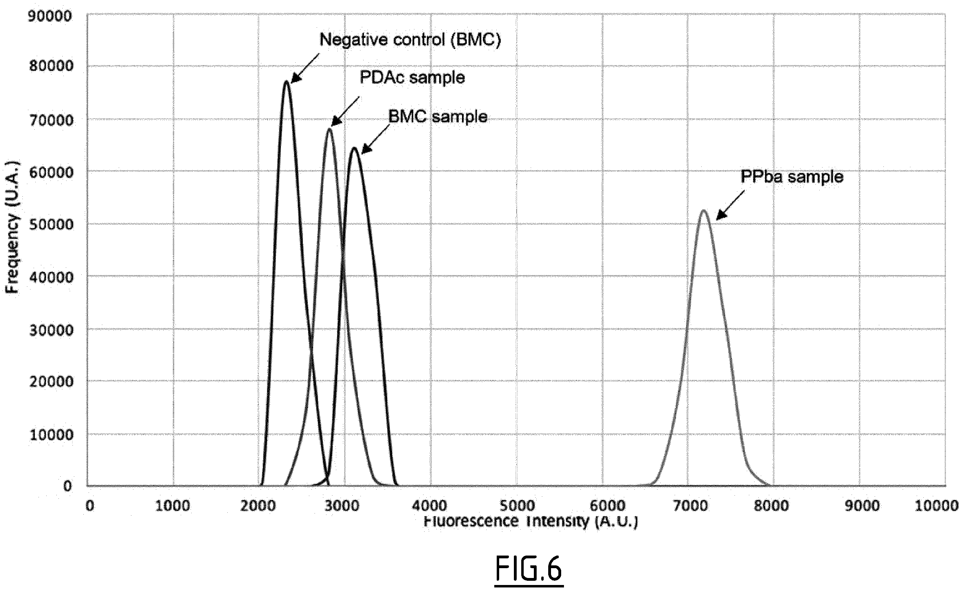

**Figure 6****:** Median fluorescent intensity of BMC negative control, BMC, PDA2 and PPba13 samples. The median fluorescence intensity of PPba13 sample is higher to the other samples.

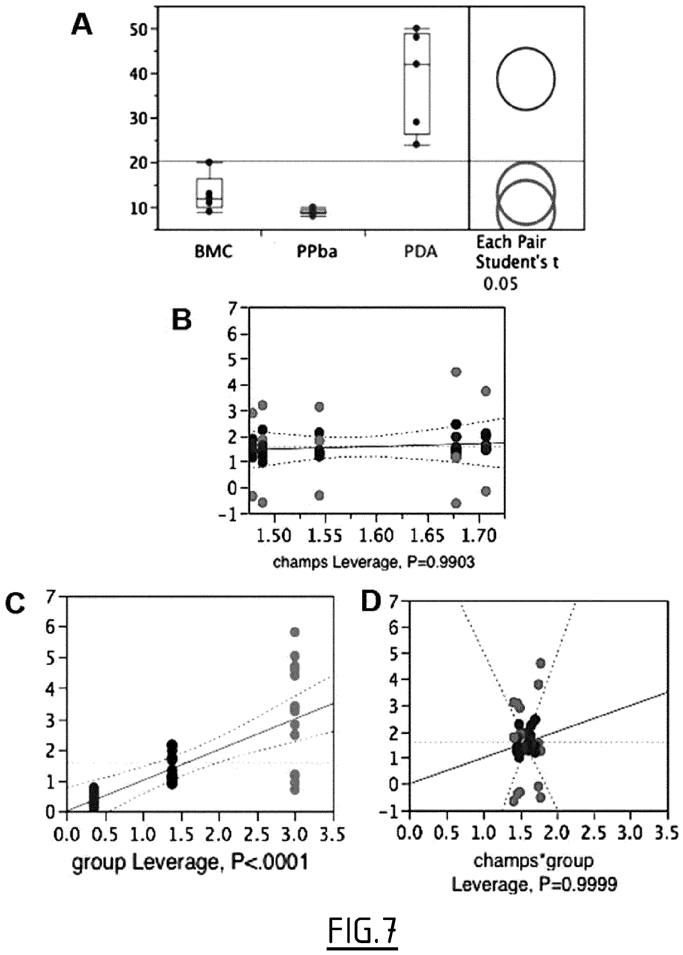

**Figure 7****:** Medians comparison for negative controls (A) ; One-Way ANOVA analysis (B, C, D) : Champ effect (B), group effect (C) and champs x groups (D) for signal/noise ratio (Leverage residuals) in ordinate. BMC are represented in red, PPba13 in green, and PDA2 in blue. The PDA2 background noise is significantly higher to the others sets. There is only a group effect. (Ordinate for A: median

### Examples

### Materials and methods

### Materials and chemicals

Cobalt-chromium (L605) disks (4,8 mm diameter, 0.25mm height, polished on one side) were obtained from Goodfellow (Lille, France). Their composition is described below (Table 1).

**Table 1 - L605 cobalt-chromium chemical composition**

| Element | Co | Cr | W | Ni | Fe | Mn |
|---|---|---|---|---|---|---|
| Chemical composition (%m) | 50 | 20 | 15 | 10 | 3,0 | 2,0 |

Tris(hydroxymethyl)aminomethane (Tris) buffer (CAS N° 77-86-1, 10 mM, pH adjusted to 8,5) and Sodium bicarbonate buffer (CAS N° 144-55-8, 100 mM, pH = 8,3) were filtered with a 250 mL sterile filter system (polyether sulfone membrane, 0,22 µm pore size, Corning) prior to their use. Ethanol (96% pure) and acetone (99,8%, AnalaR NORMAPUR^{®}) were purchased from VWR Prolabo Chemicals. Dopamine powder (Dopamine hypochloride, Alfa Aesar, A11136, 99% pure), ε-(Digoxigenin-3-0-acetamido)caproic acid N-hydroxysuccinimide ester (DIG NHS ester, 55865-5MG-F), polyethylene glycol bis-amine (PEG bis-amine, P9906) were purchased from Sigma-Aldrich. Antifading agent (Pro Long^{™} Gold), carboxyfluorescein succinimidyl ester (CFSE, C34554), and SYTOX Green (S7020) were purchased from Thermo Fischer Scientific.

### Determination of an optimal copolymer for L605 alloy coating

### (i) Determination of the copolymer optimal initial concentration of dopamine

### Disks preparation

Each disk was marked with a diamond point to identify the face in contact with the well bottom at each step. The marked disks were ultrasonically polished (VWR, Ultrasonic Cleaner) in three successive baths (acetone, ethanol and finally distilled water), 10 minutes for each bath, in order to remove oxidation and organic residues. Next the disks were separated in 3 groups: untouched (Bare Metal Controls, BMC), coated with polydopamine (PDA Controls, PDAc) and copolymers samples (PDA + PEG bis amine, PPba). After the polishing step, BMC were placed in distilled and filtered water and left under a sterile culture hood, to avoid contamination. The other disks were washed 3 times in Tris during 3 minutes under stirring onto an orbital shaker (Grant-Bio, PMS-1000i) at 400 rpm.

### Surface modification by coating

Disks in the PDAc group were immersed in dopamine only solution (1 or 2mg/ml) in Tris buffer, whereas PPba disks were immersed in a mixture of dopamine (fixed concentration: 1 mg/mL) and PEG bis-amine solution (concentration varying from 1 to 6 mg/mL) in Tris buffer. Polymerization was allowed by incubation of individual disks in the wells of a sterile and transparent polystyrene 48-wells microplate (under stirring at 400 rpm, in the dark, at room temperature) during 17 hours. Polymerization was stopped by repeated washes in clean distilled water under stirring (400 rpm, 3 minutes each), and a brief sonication (limited to 15 seconds to avoid damaging the coatings), after the last wash.

### Analysis of NHS-ester binding by CFSE

For each group (BMC, PDAc, PPba), 3 disks were immersed in a CFSE solution (50 µg/mL in sodium boicarbonate) for 30 minutes at room temperature under stirring (400 rpm), in the dark. The reaction was stopped by washing two times with distilled water (5 minutes each, under stirring at 400 rpm) and the disks were placed, marked face up, onto 20 µL of antifading mounting medium on the bottom of individual wells of 12-wells-lbidi^{®} slides (Ibidi, Germany) and left untouched for at least 30 minutes in the dark (to allow the antifade mounting medium to polymerize and render the observation by inversed fluorescent microscope possible.

### Fluorescence microscope analysis

Digital images were acquired on the coated face of each disk in the green fluorescent channel (ex 475/40 em 530/50 nm) of an Axio Observer^{®} microscope (Zeiss, Germany). The time of exposure was set on the BMC sample that had not been contacted with CFSE (background fluorescence). A second background noise control was obtained by the emission in the near infrared light channel (ex 640/30 em 690/50). The fluorescence intensity histograms from both channels were calculated using the proprietary software (ZEN^{®}, Zeiss, Germany) and raw data were exported and further analyzed using the software *Microsoft^{®} Excel.*

### (ii) Evaluation of chromatin adhesive properties of DIG-coated disks by fluorescence microscopy and SYTOX Green

### Determination of optimal conditions for maximal DIG NHS ester coating

DIG NHS ester was diluted in cascade by a 1:2 factor, in either PBS 1X or sodium bicarbonate (10mM, pH 8.3), from 500 µg/mL to 16,625 µg/mL. PPba disks were immersed individual solutions during 30 minutes, under stirring (400 rpm) at room temperature and in the dark, as for the CFSE ester. The reaction was stopped by washing three times with distilled water (5 minutes each) under stirring. DIG that had effectively onto the disks was detected by indirect immunofluorescence, using a biotynilated anti-DIG NHS ester antibody (NBP2-31191B, Novusbio, USA) and streptavidin PE (*554061BD Biosciences, USA*) as well as by chromogenic development and absorbance detection, using streptavidin-HRP (557630, BD Biosciences, USA) and TMB (*T0440, Sigma*). Background signal was set on disks that had been contacted with the buffer alone, in the absence of DIG-NHS). Absorbance was analyzed on the supernatant, on a Infinite^{®} 200 Pro plate reader (TECAN, Switzerland) whereas the amount and distribution of PE at the surface of the washed disks was analyzed by fluorescence microscopy on Ibidi 8 plates, as previously described, in the orange channel (ex 545/25, em 605/710 nm).

### Genetic material extraction

Genetic material (genomic DNA and associated proteins) derived from laboratory mouse tails and was obtained using the "F-355L DNA Release" kit (Thermo Scientific). Since the binding of digoxigenin to chromatin is thought to occur via its interaction with topoisomerase, we assessed the presence of proteins associated with the extracted DNA, using the kit "Pierce^{™} BCA Protein Assay Kit" from Thermo Scientific, prior to using this material.

### Functional analysis of DNA binding onto coated disks

Disks from the three groups were coated in optimal DIG-NHS conditions (125 µg/mL in sodium bicarbonate buffer), washed three times in distilled water and immersed in a solution of mouse tail-derived genetic material (diluted 1:10 in PBS 1X corresponding to a final concentration of 87,3 µg/mL total protein, as determined using the Pierce^{™} BCA protein assay kit, Thermo Scientific). The incubation with genetic material was carried out at 37°C and lasted 5 minutes (to mimic the time of contact between the stent retrievers and the clot in stroke patients). The disks were then thoroughly rinsed and immersed in SYTOX Green (167 nM in buffer) for 30 minutes and washed three time with distilled water. Finally, the disks were placed, marked face up, onto anti-fading mounting medium at the bottom of individual wells in a 8-wells Ibidi^{®} slide and analyzed in the green channel.

### Artificial thrombi

Artificial thrombi were generated using peripheral human whole blood, collected in dry tubes and stimulated with PMA to generate Nets in order to obtain thrombi containing extracellular Chromatin;

Fresh thrombi were retrieved from patients with stroke.

### Results and discussion

### Presence of chromatin at the thrombus surface

The presence of chromatin was evaluated at the thrombus surface and in its inside (after its sectioning), by epifluorescence microscopy. The analyzed whole thrombus were retrieved from a cerebral artery in stroke patient.

A dense mesh of chromatin was present in all the analyzed fresh thrombi (n=5) (*data not shown*).

### Determination of an intermediate polymer for coating L605 alloy with NHS ester molecules

It was first used a two-step dip-coating using PDA in the first bath and PEGba in a subsequent bath. This strategy was intended to orient the grafting of PEGba by one of its amine arms and leave the other amine group available for the subsequent reaction with the NHS ester, in order to drive a unidirectional binding with DIG instead of having disordered bindings. The concentration of PDA at 2 mg/ml was based on the most widely used. The concentration of PEGba was set to 6 mg/mL, to reproduce the ratio 3:1 that had previously been reported as ideal in combination with another self-assembling polymer. Contrary to what expected, the grafting of the CFSE NHS ester was higher onto PDA alone than on PDA + PEGba (*see* *Figure 2*), suggesting that the both of the amines of the PEGba had reacted with the cathecols of the PDA polymer and none was left free and available to react with the NHS ester in the subsequent step.

The strategy was therefore changes and PEGba and PDA were used in the same polymerization bath: the excess in amine groups was supposed to allow the formation of a copolymer with free amines functions at its surface, available for the subsequent reaction with the NHS ester. A copolymer made using the same concentration of the two reagents (2 mg/mL of initial dopamine and 6 mg/mL of PEGba) was compared to PDA alone at 2mg /ml, BMC, and various concentrations of PEGba used in combination with a fixed concentration of PDA at1 mg/mL (*see* *Figure 2*). The results show that the use of PDA 1mg/ml in combination with PEGba 3mg/ml during 40h gives the best copolymer condition for subsequent grafting of a NHS ester (*CFSE in the case of* *Figure 2*). This condition has therefore been chosen as the working copolymer solution for the subsequent studies.

### Determination of the concentration and buffer for DIG NHS ester grafting

Five different concentrations (cascade 1:2 dilutions) of DIG-NHS ester, ranging from 31.25 to 500 µg/ml, prepared in either sodium bicarbonate (100mM, pH 8.3) or PBS (pH 7.4) were evaluated. The effective grafting of DIG NHS ester onto the PPba copolymer was evaluated using indirect immunofluorescence and an antibody specifically directed against the DIG NHS ester.

As shown in Figure 3, the highest disk coverage with DIG, as detected by immunofluorescence intensity, was obtained at the concentration of 125 µg/mL. Both sodium bicarbonate and PBS buffer were effective, but the fluorescence curve obtained with PBS 1X, although yielding a higher peak, was wider than the one obtained with sodium bicarbonate, meaning the density of DIG NHS ester onto the copolymer was more heterogeneous. Sodium bicarbonate and a concentration of 125µg/ml of DIG NHS was therefore retained for the subsequent functional testing with genetic material.

### Evaluation of chromatin adhesive properties of DIG-coated disks by fluorescence microscopy and SYTOX Green

PPba (1:3) disks were immersed in 125µg/ml DIG NHS ester solution in bicarbonate sodium buffer, rinsed and contacted during 5 minutes at 37°C with the genetic material (obtained by extraction form the tail of laboratory mice, diluted in PBS 1X; final protein concentration set to 87,3 µg/mL). The experimental samples were then rinsed and the binding of DNA revealed by SYTOX Green. BMC and PDA-coated disks served as control for specific binding to DIG (vs passive adsorption on the surface).

The fluorescence signal obtained at 5X magnification from the different samples was very large (the curves do not display a narrow "peak" but rather a spread along the X-axis) suggesting that the presence of DNA was globally inhomogeneous (*see* *Figure 4*).

The fluorescence of SYTOX green was assessed at higher magnification (x20) in 5 random fields, on each disk. As shown in Figure 5, one-way ANOVA analysis ruled out the inhomogeneity of DNA binding as there was no "champs" (field) effect (A, champs leverage, p=NS) in any of the groups (C, champs*group Leverage, P=NS). In contrast, the higher magnification analysis, possibly thanks to the more consistent fluorescence signal, showed that the binding of DNA, as detected by SYTOX green signal, was highest on the disks coated with PPba (green) as compared to those coated with PDA (Blue) or the bare metal controls (BMS Red). These data demonstrate that the affinity binding of DNA through DIG-coated surfaces is consistently superior to what can be achieved with the passive adsorption of genetic material onto the stent-compatible alloy surfaces (panel B, group leverage, p<0.0001).

The superiority of functionalized surfaces vs bare metal and control coated surfaces is clear in Figure 6, which shows a representative fluorescent curve in the SYTOX green channel to compare each group

However, the use of fluorescence as a reading out for the functional studies could be biased by the background noise (fluorescence of the negative control in the SYTOX green channel) yielded by the aromatic ring of dopamine, as reflected by the higher background detected in the PDA-coated samples (Median Fluorescence Intensity=38.6 in PDA vs 13 in BMC, Figure 7 A, p<0.001). However, PDA was also contained in the copolymer but its concentration was reduced by half and this might explain why the median background fluorescence intensity observed in PPba samples is very low and not different from BMC controls (*see* *Figure 7A*). When using the signal/noise ratio (fluorescence in the experimental disks / fluorescence in the control disks) for comparing data between groups, the statistical analysis indicates a group effect *(see* *Figure 6***Figure** *C*, p<0.001) at least as strong as the one observed by comparing absolute signal values from experimental disks (Figure) where the median fluorescence intensity in PDA was likely biased by the background noise (contrary to BMC and PPba disks).

### Coating on nitinol

The protocol used for cobalt-chromimum disked was used for the coating of disks made of nitinol (another stent-suitable alloy) and the performance of digoxigenin-coated surface in terms of capture of soluble DNA was confirmed (*data not shown*).

### Coating of distamicvn azide

The coating with distamicyn azide has been produced by a copper-free chemistry protocol.

### Evaluation of chromatin adhesive properties of DIG-coated disks and Distamicin-Azide coated disks

The ability of fragments of clot retrieval devices coated with digoxigenin-NHS vs those coated with Distamicin-Azide vs bare metal disks to capture DNA and protein material from artificial (*in vitro*) and from patient's (*ex-vivo*) thrombi was then evaluated.

To this aim, the tested devices (n=>3 / group) were inserted manually in fragments of the same thrombus and hold still within a 3D printed recipient immersed in human plasma (5 minutes, at 37°C). The tested devices were then removed manually, incubated with DAPI 1µg/ml and Evan's Blue 1% in PBS for 5 minutes. After careful washing with PBS, the tested devices were mounted using Prolong Gold anti-fade mounting medium in glass bottom dishes and the presence of thrombus captured at their surface was examined by fluorescence microscopy.

Digoxigenin and Distamycin coated devices appeared to be effective for improving the capture of thrombus material as compared with uncoated (bare metal) devices (*data not shown*). The experiments have been repeated at least three times, yielding similar results (qualitative analysis only).

### Conclusion

A coating comprising digoxigenin or distamycin specifically confers the ability to bind genetic material onto stent-compatible metal surfaces. Distamycin and Digoxigenin coated struts both improve the capture of patient's thrombus material as compared with uncoated struts. This bioactive coating is thus suitable for improving the performances of clot retrieval devices.

Besides, the material used for the coating is natural, biocompatible and easily available, and is thus suitable for production at the industrial scale.

## Claims

1. Clot retrieval device, wherein at least the part of said device intended to be in contact with a clot is coated with at least one chromatin binding agent, wherein said chromatin binding agent is selected from the group consisting of digoxigenin, a digoxigenin derivative, distamycin and a distamycin derivative, and wherein said chromatin binding agent is coated on the part of the device intended to be in contact with a clot via at least one coating agent.

2. Clot retrieval device according to claim 1, wherein said clot retrieval device is a stent retriever.

3. Clot retrieval device according to claim 1 or 2, wherein said coating agent is polydopamine (PDA) or PDA-PEG bis amine.

4. Clot retrieval device according to any one of claims 1 to 3, wherein at least the part of said device intended to be in contact with a clot is coated with at least one coating agent and said chromatin binding agent is linked to said coating agent.

5. Clot retrieval device according to any one of claims 1 to 4, wherein the digoxigenin derivative is a compound of the following formula (III) 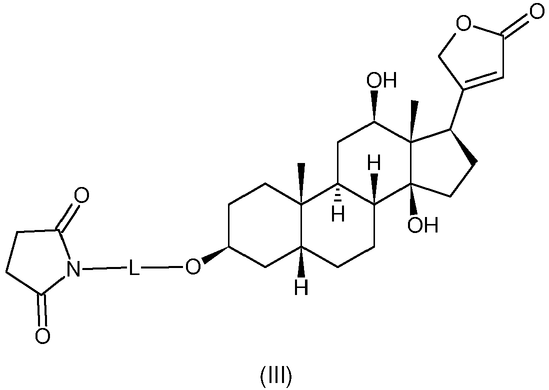 wherein L is a linker comprising at least one group selected from the group consisting of an amino, ester, amido, carboxy and hydroxyl group.

6. Clot retrieval device according to any one of claim 1 to 5, wherein the digoxigenin derivative is digoxigenin NHS ester and/or the distamycin derivative is distamycin azide.

7. Clot retrieval device according to any one of claims 1 to 6, wherein (i) the chromatin binding agent is digoxigenin NHS ester and the coating agent is PDA PEG bis-amine or (ii) the chromatin binding agent is distamycin azide and the coating agent is PDA.

8. Method for producing a clot retrieval device according to any one of claims 1 to 7, wherein said method comprises:
a) coating at least the part of the device intended to be in contact with a clot with at least one coating agent, and
b) contacting the part of the device coated with at least one coating agent obtained in step a) with at least one chromatin binding agent.

9. Method according to claim 8, wherein step a) comprises contacting at least the part of the device intended to be in contact with a clot with a solution comprising dopamine and, optionally, PEG bis-amine.

10. Use of at least one chromatin binding agent for manufacturing a clot retrieval device according to any one of claims 1 to 7, wherein said chromatin binding agent is selected from the group consisting of digoxigenin, a digoxigenin derivative, distamycin and a distamycin derivative.

## Patentansprüche

1. Gerinnselentnahmevorrichtung, wobei mindestens der Teil der Vorrichtung, der mit einem Gerinnsel in Kontakt kommen soll, mit mindestens einem Chromatin-Bindemittel beschichtet ist, wobei das Chromatin-Bindemittel ausgewählt ist aus der Gruppe, bestehend aus Digoxigenin, einem Digoxigenin-Derivat, Distamycin und einem Distamycin-Derivat, und wobei das Chromatin-Bindemittel über mindestens ein Beschichtungsmittel auf den Teil der Vorrichtung beschichtet ist, der mit einem Gerinnsel in Kontakt sein soll.

2. Gerinnselentnahmevorrichtung nach Anspruch 1, wobei die Gerinnselentnahmevorrichtung eine Stent-Entnahmeeinrichtung ist.

3. Gerinnselentnahmevorrichtung nach Anspruch 1 oder 2, wobei das Beschichtungsmittel Polydopamin (PDA) oder PDA-PEG-bis-Amin ist.

4. Gerinnselentnahmevorrichtung nach einem der Ansprüche 1 bis 3, wobei zumindest der Teil der Vorrichtung, der mit einem Gerinnsel in Kontakt kommen soll, mit mindestens einem Beschichtungsmittel beschichtet ist und das Chromatin-Bindemittel mit dem Beschichtungsmittel verbunden ist.

5. Gerinnselentnahmevorrichtung nach einem der Ansprüche 1 bis 4, wobei das Digoxigenin-Derivat eine Verbindung der folgenden Formel (III) ist 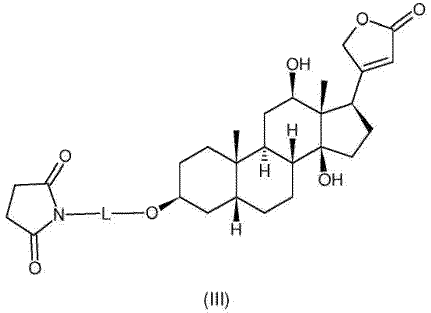 worin L ein Linker ist, umfassend mindestens eine Gruppe, die ausgewählt ist aus der Gruppe, bestehend aus einer Amino-, Ester-, Amido-, Carboxy- und Hydroxylgruppe.

6. Gerinnselentnahmevorrichtung nach einem der Ansprüche 1 bis 5, wobei das Digoxigenin-Derivat Digoxigenin-NHS-Ester und/oder das Distamycin-Derivat Distamycin-Azid ist.

7. Gerinnselentnahmevorrichtung nach einem der Ansprüche 1 bis 6, wobei (i) das Chromatin-Bindemittel Digoxigenin-NHS-Ester und das Beschichtungsmittel PDA-PEG-Bis-Amin ist oder (ii) das Chromatin-Bindemittel Distamycin-Azid und das Beschichtungsmittel PDA ist.

8. Verfahren zum Herstellen einer Gerinnselentnahmevorrichtung nach einem der Ansprüche 1 bis 7, wobei das Verfahren Folgendes umfasst:
a) Beschichten mindestens des Teils der Vorrichtung, der mit einem Gerinnsel in Kontakt kommen soll, mit mindestens einem Beschichtungsmittel, und
b) Inkontaktbringen des Teils der Vorrichtung, der mit mindestens einem Beschichtungsmittel beschichtet ist, das in Schritt a) erlangt wird, mit mindestens einem Chromatin-Bindemittel.

9. Verfahren nach Anspruch 8, wobei Schritt a) ein Inkontaktbringen mindestens des Teils der Vorrichtung, der mit einem Gerinnsel in Kontakt kommen soll, mit einer Lösung umfasst, umfassend Dopamin und optional PEG-bis-Amin.

10. Verwendung mindestens eines Chromatinbindemittels zur Herstellung einer Gerinnselentnahmevorrichtung nach einem der Ansprüche 1 bis 7, wobei das Chromatinbindemittel ausgewählt ist aus der Gruppe, bestehend aus Digoxigenin, einem Digoxigenin-Derivat, Distamycin und einem Distamycin-Derivat.

## Revendications

1. Dispositif d'extraction de caillot, dans lequel au moins la partie dudit dispositif destinée à être en contact avec un caillot est recouverte d'au moins un agent se liant à la chromatine, dans lequel ledit agent se liant à la chromatine est choisi dans le groupe constitué de la digoxigénine, d'un dérivé de digoxigénine, de la distamycine et d'un dérivé de distamycine, et dans lequel ledit agent se liant à la chromatine est revêtu sur la partie du dispositif destinée à être en contact avec un caillot par l'intermédiaire d'au moins un agent de revêtement.

2. Dispositif d'extraction de caillot selon la revendication 1, ledit dispositif d'extraction de caillot étant un extracteur de stent.

3. Dispositif d'extraction de caillot selon la revendication 1 ou 2, dans lequel ledit agent d'enrobage est la polydopamine (PDA) ou PDA-PEG-bis-amine.

4. Dispositif d'extraction de caillot selon l'une quelconque des revendications 1 à 3, dans lequel au moins la partie dudit dispositif destinée à être en contact avec un caillot est revêtue d'au moins un agent d'enrobage et ledit agent se liant à la chromatine est lié audit agent de revêtement.

5. Dispositif d'extraction de caillot selon l'une quelconque des revendications 1 à 4, dans lequel le dérivé de digoxigénine est un composé de la formule (III) suivante 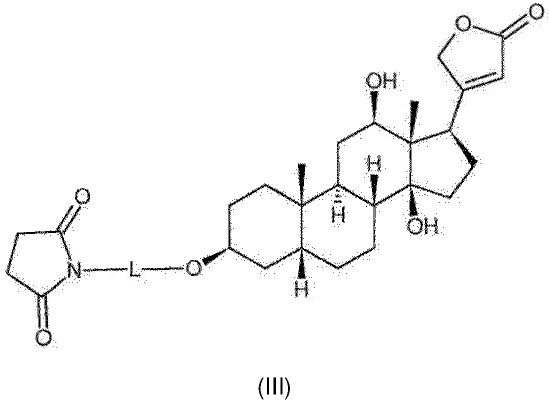 dans laquelle L est un lieur comprenant au moins un groupe choisi dans le groupe constitué d'un groupe amino, ester, amido, carboxy et hydroxyle.

6. Dispositif d'extraction de caillot selon l'une quelconque des revendications 1 à 5, dans lequel le dérivé de digoxigénine est l'ester NHS de digoxigénine et/ou le dérivé de distamycine est l'azoture de distamycine.

7. Dispositif d'extraction de caillot selon l'une quelconque des revendications 1 à 6, dans lequel (i) l'agent se liant à la chromatine est l'ester NHS de digoxigénine et l'agent de revêtement est PDA PEG-bis-amine ou (ii) l'agent se liant à la chromatine est l'azoture de distamycine et l'agent de revêtement est PDA.

8. Procédé de production d'un dispositif d'extraction de caillot selon l'une quelconque des revendications 1 à 7, ledit procédé comprenant :
a) le revêtement d'au moins la partie du dispositif destinée à être en contact avec un caillot avec au moins un agent de revêtement, et
b) la mise en contact de la partie du dispositif revêtue d'au moins un agent de revêtement obtenue à l'étape a) avec au moins un agent se liant à la chromatine.

9. Procédé selon la revendication 8, dans lequel l'étape a) comprend la mise en contact d'au moins la partie du dispositif destinée à être en contact avec un caillot avec une solution comprenant de la dopamine et, éventuellement, PEG-bis-amine.

10. Utilisation d'au moins un agent se liant à la chromatine pour fabriquer un dispositif d'extraction de caillot selon l'une quelconque des revendications 1 à 7, ledit agent se liant à la chromatine étant choisi dans le groupe constitué de la digoxigénine, d'un dérivé de digoxigénine, de la distamycine et d'un dérivé de distamycine.
